# EUROPEAN PATENT SPECIFICATION

(11) **EP 3 136 974 B1**
(45) Date of publication and mention of the grant of the patent: **19.12.2018**
(21) Application number: 14736018.4
(22) Date of filing: 02.05.2014
(51) Int. Cl.: A61B 8/08, A61B 8/00, A61B 5/00

(54) **ELASTOGRAPHY VISUALIZATION**
ELASTOGRAFIEVISUALISIERUNG
VISUALISATION D'ÉLASTOGRAPHIE

(43) Date of publication of application: 08.03.2017
(73) Proprietor: B-K Medical ApS, 2730 Herlev (DK)
(72) Inventor: AZAR, Reza, Vancouver, British Columbia (CA); DICKIE, Kris, Vancouver, British Columbia (CA); ALEXANDER, Michelle, Lithia Springs, Georgia (US)
(74) Representative: Gevers Patents
(86) International application number: PCT/IB2014/061165
(87) International publication number: WO 2015/166311

(56) References cited:
- EP-A1- 1 889 571
- WO-A1-2008/132504

## Description

### TECHNICAL FIELD

The following generally relates to elastography visualization and more particularly to ultrasound elastography image visualization, and is described with particular application to an ultrasound imaging system.

### BACKGROUND

An ultrasound imaging system has included at least an ultrasound probe and a console. The ultrasound probe houses a transducer array of transducing elements, and the console includes a display monitor and a user interface. The transducing elements transmit an ultrasound signal into a field of view and receive echoes produced in response to the signal interacting with structure therein. In B-mode, the echoes are processed, producing a sequence of focused, coherent echo samples along focused scanlines of a scanplane. The scanlines are scan converted into a format of a display monitor and visually presented as image via the display monitor.

With ultrasound elastography imaging, or real-time strain imaging, ultrasound images are acquired from the tissue while the tissue undergoes compression or deformation. The compression or deformation can be applied manually by the user (i.e. by slightly pressing the transducer against the tissue), induced by internal tissue motions (due to breathing or heart beat), or induced through focused beams of ultrasound energy to produce movement. During the compression cycle, ultrasound signals are acquired and processed to generate the corresponding strain images. The strain images have been displayed, for example, as a measure of tissue elasticity along-side the B-mode images.

Strain images, generally, are a function of the applied compression. As a consequence, small compressions may not generate enough contrast and will lower the detectability of the tissue abnormalities, while large compressions may result in unreliable measurements and invalid images. In addition to the compression, strain images are also a function of underlying tissue structure, as well as their ultrasonic signal to noise ratio. As such, some of the displayed pixels may not represent valid or useful information. Unfortunately, this may result in a false interpretation of the image.

EP-A-1889571 discloses an ultrasonic diagnostic apparatus in which allowance for modulus of elasticity is provided. The apparatus comprise a computing section for determining a thickness change waveform which represents a variation in distance between two arbitrary measuring points on a subject based on received echo signals and a reference waveform, internal information of the subject being obtained by comparing the thickness change waveform and the reference waveform to one another.

### SUMMARY

Aspects of the application address the above matters, and others.

In one aspect, a method according to claim 1 is provided.

In another aspect, a system according to claim 12 is provided.

Those skilled in the art will recognize still other aspects of the present application upon reading and understanding the attached description.

### BRIEF DESCRIPTION OF THE DRAWINGS

The application is illustrated by way of example and not limitation in the figures of the accompanying drawings, in which like references indicate similar elements and in which:
Figure 1 schematically illustrates an example ultrasound imaging system with an elastogram processor and a rendering engine;
Figure 2 schematically illustrates an example of the elastogram processor;
Figure 3 schematically illustrates an example of the rendering engine;
Figure 4 shows an example B-mode image;
Figure 5 shows an example strain image;
Figure 6 shows an example of the strain image of Figure 5 superimposed over the B-mode image of Figure 4;
Figure 7 shows an example of a soft blend strain image;
Figure 8 shows an example of the soft blend strain image of Figure 7 superimposed over the B-mode image of Figure 4;
Figure 9 shows an example of a hard blend strain image;
Figure 10 shows an example of the hard blend strain image of Figure 9 superimposed over the B-mode image of Figure 4;
Figure 11 shows an example in which strain image pixels corresponding to B-mode image pixels generated with not enough signal are not displayed or are displayed completely transparent over the B-mode image of Figure 4;
Figure 12 illustrates an example method in accordance with the embodiments disclosed herein;
Figure 13 illustrates example ultrasound imaging system in accordance with the embodiments disclosed herein; and
Figure 14 illustrates another example ultrasound imaging system in accordance with the embodiments disclosed herein.

### DETAILED DESCRIPTION

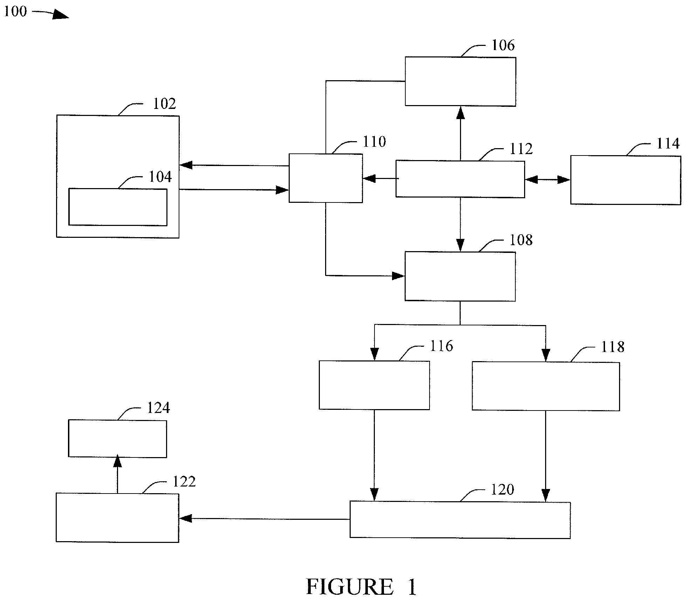

Figure 1 illustrates an example imaging system 100, such as an ultrasound imaging system.

The ultrasound imaging system 100 includes a transducer array 102. The transducer array 102 can include a one dimensional (1D) or two dimensional (2D) array of transducer elements 104. The transducer elements 104 are configured to transmit ultrasound signals and receive echo signals. Suitable arrays 102 include linear, curved, and/or otherwise shaped. The transducer array 102 can be fully populated or sparse.

The ultrasound imaging system 100 includes transmit circuitry 106. The transmit circuitry 106 generates a set of radio frequency (RF) pulses that are conveyed to the transducer array 102. The set of pulses actuates a corresponding set of the transducer elements 104, causing the elements to transmit ultrasound signals into an examination or scan field of view. For elastography imaging, compression/deformation is applied manually by the user, through focused beams, induced internally by heartbeat or breathing, etc. during transmit of ultrasound signals.

The ultrasound imaging system 100 includes receive circuitry 108. The receive circuitry 108 receives echoes (RF signals) generated in response to the transmitted ultrasound signals from the transducer array 102. The echoes, generally, are a result of the interaction between the emitted ultrasound signals and the structure (e.g., flowing blood cells, organ cells, etc.) in the scan field of view. For elastography imaging, frames are acquired continuously at a rate of up to 30 frames per second or higher.

The ultrasound imaging system 100 includes a switch 110. The switch 110 switches between the transmit circuitry 106 and the receive circuitry 108, depending on whether the transducer array 102 is operated in transmit or receive mode. In transmit mode, the switch 110 electrically connects the transmit circuitry 106 to the elements 104. In receive mode, the switch 110 electrically connects the receive circuitry 108 to the elements 104.

The ultrasound imaging system 100 includes a controller 112. The controller 112 controls one or more of the transmit circuitry 106, the receive circuitry 108 or the switch 110. Such control can be based on available modes of operation. Examples of such modes of operation include one or more of B-mode, elastography mode, A-mode, velocity flow mode, Doppler mode, etc.

The ultrasound imaging system 100 includes a user interface (UI) 114. The UI 114 may include one or more input devices (e.g., a button, a knob, a slider, a touch pad, etc.) and/or one or more output devices (e.g., a display screen, lights, a speaker, etc.). The UI 114 can be used to select an imaging mode, activate scanning, etc.

The ultrasound imaging system 100 further includes an echo processor 116 that processes received echoes. Such processing may include applying time delays, weighting on the channels, summing, and/or otherwise beamforming received echoes. In B-mode, the echo processor 116 produces a sequence of focused, coherent echo samples along focused scanlines of a scanplane. Other processing may lower speckle, improve specular reflector delineation, and/or includes FIR filtering, IIR filtering, etc.

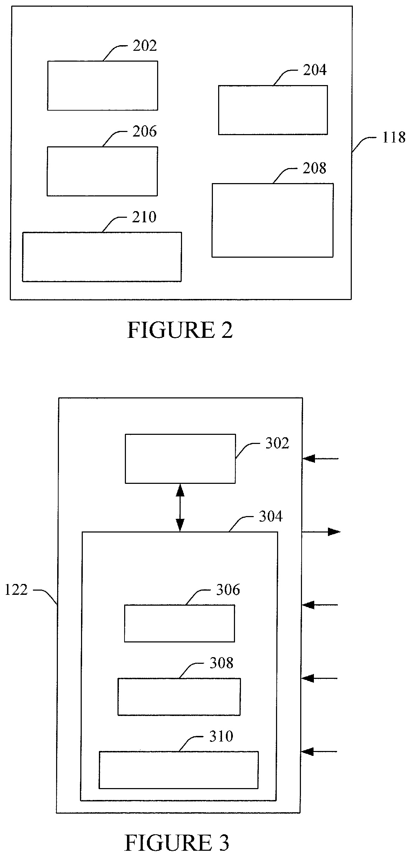

The elastogram processor 118 processes the received signals and generates a strain image between at least two sequential frames, and a corresponding correlation image(s). In a variation, the elastogram processor 118 processes the B-mode images from the echo processor 116 and generates the strain image and the corresponding correlation image. Briefly turning to Figure 2, a non-limiting example of the elastogram processor 118 is schematically illustrated.

The elastogram processor 118 includes a motion estimator 202. The motion estimator 202 estimates motion between sequences of received signals. In one non-limiting instance, this includes dividing the signals into windows of small overlapping windows and applying a motion tracking algorithm to each window. Elastograms with different resolutions can be generated by adjusting the size and overlap between these windows. Known and/or other motion algorithms can be employed. The motion estimator 202 outputs a displacement image.

The motion estimator 202 also outputs a corresponding correlation image. The displacement image represents the displacement between successive windows, and correlation image indicates a degree of match or similarity between the corresponding windows. In a non-limiting example, the normalized correlation image includes values between minus one (-1) and one (1), which indicates reliable sub-portions of the displacement image (e.g. sub-portions corresponding to higher correlation values) and less reliable and/or unreliable sub-portions of the displacement image (e.g. sub-portions corresponding to lower correlation values). Generally, -1 means the windows are inverted version of each other, 0 means no similarity, and 1 means they are perfect match. It is understood that alternative measures of similarity such as non-normalized correlation image, sum of absolute differences, sum of absolute differences etc. as well as techniques that use phase shift estimation and complex correlation are contemplated herein.

The elastogram processor 118 further includes a spatial filter 204. The spatial filter 204 applies a 2D spatial filtering to the displacement image and the correlation image. Suitable filters include a 2D medial filterer, a 2D mean filterer, and/or other filter. The 2D medial filterer, for example, removes outliers. The 2D mean filterer, for example, improves the signal to noise ratio (SNR). In a variation, the spatial filter 204 is omitted from the elastogram processor 118.

The elastogram processor 118 further includes a strain estimator 206. The strain estimator 206 processes the displacement images and generates strain images. The strain estimator 206 can employ known and/or other strain estimation algorithms. An example of a known strain estimation algorithm includes a least-squares strain estimator.

The elastogram processor 118 further includes spatial and temporal filter 208. The spatial and temporal filter 208 applies spatial filtering and temporal persistency to both the strain images and the correlation images. This, for example, improves the SNR of both the strain images and the correlation images. The spatial and temporal filter 208 can employ known and/or other strain estimation algorithms. In a variation, the spatial and temporal filter 208 is omitted from the elastogram processor 118.

The elastogram processor 118 further includes a dynamic range adjuster 210. The dynamic range adjuster 210 maps the strain values a predetermined scale such as the full scale other scale. In one instance, the mapping maintains the maximum dynamic range of the strain values. Examples of suitable algorithms include contrast stretching, histogram equalization, and/or other algorithms.

Returning to Figure 1, the ultrasound imaging system 100 further includes a scan converter 120. The scan converter 120 scan converts the output of the echo processor 116 and generates data for display, for example, by converting the data to the coordinate system of a display. The scan converter 120 also scan converts both the strain image and correlation images based on the geometry of the B-mode image. In a variation, a different scan converter is used for the B-mode image and the strain and correlation images.

The ultrasound imaging system 100 includes a rendering engine 122 and a display 124. The rendering engine 122 visually presents the B-mode image overlaid with the strain image. In one instance, the rendering engine 122 overlays only the strain image (e.g., a color-coded or gray scale) over the B-mode image. In this instance, the strain image is a 1D map that maps the elasticity or strain values directly to the B-mode image.

In another instance, the rendering engine 122 creates a 2D overlay image based on the strain image and the correlation image and/or the B-mode image. As described in greater detail below, the overlay image takes into account the reliability of each pixel of the strain image and gradually or abruptly visually suppresses (e.g., ignores, renders transparent, etc.) strain image pixels as a function of the reliability. This may include taking into account the signal used to create a B-mode image pixel, and visually suppressing the strain image pixel based on the reliability of the B-mode image pixel.

As such, the observer of the combined image B-mode/strain image will be apprised of whether a strain image pixel corresponds to a valid or suspect measurement, which may facilitate mitigating a false interpretation of the B-mode image. As a consequence, the displayed data can be used, for example, during the training phase when the clinicians are trying to improve their scanning techniques, during live scan to provide feedback to an end user such that the end user knows when a good sequence of elastograms have been acquired, during exam review when selecting individual frames where good images are generated, etc.

This also allows, in one non-limiting instance, the clinician to better evaluate the generated images in real-time (as the data is acquired and images are generated) and/or off-line during the exam review, relative to just displaying the strain image alone over a B-mode image. It also allows the user to improve their scanning techniques and acquire better strain images. Generally, the processing of the rendering engine 122 improves visualization of strain images. It also provides for calculation of quality feedback to help clinicians acquire repeatable and more reliable strain images.

It is to be appreciated that one or more of the echo processor 116, the elastogram processor 118, and the rendering engine 122 can be implemented via a processor (e.g., a microprocessor, central processing unit, etc.) executing one or more computer readable instructions encoded or embedded on computer readable storage medium, such as physical memory.

Turning to Figure 3, an example of the rendering engine 122 is schematically illustrated.

The rendering engine 122 includes a graphics processor 302 and a visualization algorithm memory 304. The graphics processor 302 receives, as input, a signal from the controller 112 (Figure 1). The signal indicates the visualization mode. The visualization mode can be determined based on a predetermined default mode, a user input through the UI 114 (Figure 1), and/or otherwise. The graphics processor 302 also receives one or more of the B-mode image, the strain image and/or the correlation image.

The graphics processor 302, based on the visualization mode, retrieves and/or invokes a suitable algorithm from the visualization algorithm memory 304. In the illustrated embodiment, the visualization algorithm memory 304 stores at least one of a soft blend 306 algorithm, a hard blend 308 algorithm or a B-mode priority 310 algorithm. Alternatively, the graphics processor 302 displays the B-mode image with the strain image, unmodified, overlaid there over. For this, the strain image can be considered a 1D map in that it provides only strain information, which is mapped directly to the B-mode image.

With the soft blend 306 algorithm, pixels of the strain image corresponding to lower correlation values in the correlation image are rendered more transparent, and pixels of the strain image corresponding to higher correlation values in the correlation image are rendered opaque or less transparent. In this mode, the graphics processor 302 identifies a correlation value of a pixel from the correlation image that corresponds to the strain image pixel being processed. The graphics processor 302 then identifies a transparency level for the correlation value. This can be through a look up table (LUT), a mathematical function (e.g., a polynomial), and/or otherwise. The graphics processor 302 renders the pixel with the transparency level.

The transition in transparency from a correlation value of zero (or some other value) to a correlation value of one (or some other range) can be linear, non-linear, or have both linear and non-linear regions. The transition in transparency can also be continuous, discrete or have both continuous and discrete regions. With this mode, a user sees less strain image and more the B-mode image (in the background) as the correlation value approaches zero and more strain image and less the B-mode image (in the background) as the correlation value approaches one. The soft blend strain image can be considered a 2D image in that it provides strain information and strain reliably information.

With the hard blend 308 algorithm, strain image pixels with a corresponding correlation value less than a predetermined threshold are rendered transparent, and all other pixels are rendered opaque (or less transparent). In this mode, the graphics processor 302 identifies the correlation value of a pixel from the correlation image that corresponds to the strain image pixel being processed. The graphics processor 302 the compares the correlation value with a predetermined threshold.

The graphics processor 302 then makes a binary decision as to whether to show the pixel or not (or display it transparent). The graphics processor 302 renders the pixel accordingly. This mode causes a sub-part of a strain image corresponding to low correlation to be completely removed or hidden. The hard blend strain image, like the soft blend strain image, can be considered a 2D image in that it provides strain information and strain reliably information.

With the B-mode priority 310 algorithm, the graphics processor 302 compares a B-mode image pixel value with a predetermined threshold. If the pixel value is less than the predetermined threshold, the strain image pixel corresponding to the B-mode image pixel is not displayed or is displayed transparent. Otherwise, the strain image pixel is displayed or is displayed, for example, based on the soft blend 306 algorithm, the hard blend 308 algorithm, and/or otherwise.

With this algorithm, dark regions in the B-mode image are displayed as B-mode only, with no strain overlay. This ensures that elastography data are not displayed when there is not enough of a predetermined amount of ultrasound signal. The reason for this is that the strain values in such regions may not be valid and/or reliable due to lack of signal. The B-mode priority strain image, like the soft blend strain image and the hard blend strain image, can be considered a 2D image in that it provides strain information and strain reliably information.

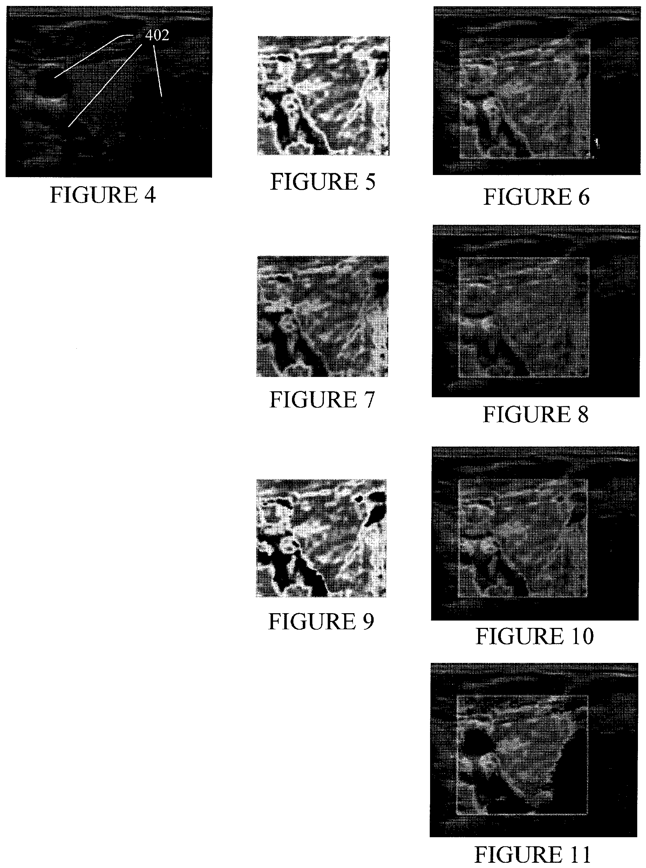

Figure 4 show an example of a B-mode image. Figure 5 shows an example of a strain image. Figure 6 shows an example of the B-mode image of Figure 4 with the strain image of Figure 5 superimposed there over. In Figure 6, the strain image is superimposed on the B-mode image, and the correlation image is ignored. With Figure 6, invalid or poor strain measurements are displayed and shown to the end user. This may result in false interpretation of the image. In this example, the strain image is shown in gray scale. However, it is to be understood that the strain image can be shown using a color map.

Figure 7 shows an example soft blend strain image. As described herein, the rendering engine 122 generates a soft blend strain image by applying a transparency map to the strain image. For this, the rendering engine 126, for a pixel in the strain image, identifies a corresponding pixel in the correlation image. A correlation value is identified for the identified pixel. A transparency level is then identified for the identified correlation value. The transparency level is then applied to the pixel in the strain image. This is repeated for all the pixels in the strain image. Figure 8 shows an example of the B-mode image of Figure 4 with the soft blend strain image of Figure 7 superimposed there over.

Figure 9 shows an example hard blend strain image. As described herein, the rendering engine 122 generates a hard blend image by thresholding the strain image. For this, the rendering engine 126, for a pixel in the strain image, identifies a corresponding pixel in the correlation image. A correlation value is identified for the identified pixel. The correlation value is compared against a predetermined threshold. If the correlation value is below the predetermined threshold, the strain image pixel value is set to completely transparent. Otherwise, the strain image pixel value is set with a transparency level from semi-transparent to opaque. This is repeated for all the pixels in the strain image. Figure 10 shows an example of the B-mode image of Figure 4 with the hard blend strain image of Figure 9 superimposed there over.

Figure 11 shows an example B-mode priority image. As described herein, the rendering engine 122 identifies, for a pixel in the B-mode image, a pixel value. The rendering engine 122 thresholds the B-mode image pixel value. For this, the rendering engine 126 compares the B-mode pixel value with a predetermined threshold. If the B-mode pixel value is below the predetermined threshold, the strain image pixel value is set to completely transparent or ignored. Otherwise, the strain image pixel value is set with a transparency level from semi-transparent to opaque.

This is repeated for all the pixels in the B-mode image and all the corresponding pixels in the strain image. Figure 11 shows an example of the B-mode image of Figure 4 with the only pixels of strain image of Figure 4 with values satisfying the threshold superimposed there over. The pixels of strain image of Figure 4 with values that do not satisfy the threshold are not superimposed there over. In the illustrated example, darker region 402 (Figure 4) represent regions of low signal, and do not satisfy the predetermined threshold, and the strain image pixels corresponding to thereto are not shown.

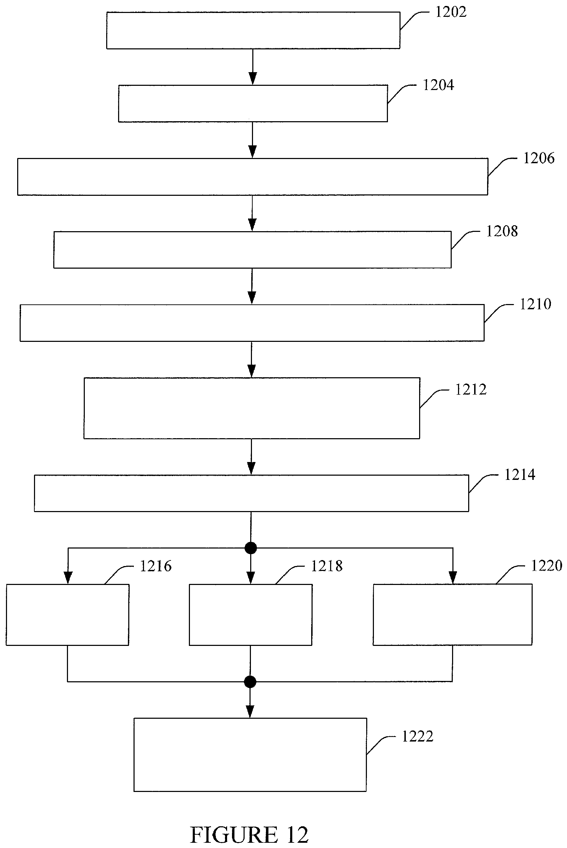

Figure 12 illustrates a method.

It is to be appreciated that the order of the following acts is provided for explanatory purposes and is not limiting. As such, one or more of the following acts may occur in a different order. Furthermore, one or more of the following acts may be omitted and/or one or more additional acts may be added.

At 1202, an array of ultrasound transducer elements is placed again a surface of a subject or object and activated to transmit an ultrasound signal into a field of view.

At 1204, a first set of echoes is received.

At 1206, pressure is applied to the subject or object. As discussed herein, this can be achieved by manually applying a pressure to the surface via the array, applying focused beams of ultrasound energy to the subject or object, etc.

At 1208, a second set of echoes (compression echoes) is received. As described herein, the second set of echoes may include two or more sets of echoes.

At 1210, the first set of echoes is processed, generating a B-mode image.

At 1212, the second set of echoes is processed, generating a strain image and a correlation image. As described herein, the strain image and corresponding correlation image(s) may be generated from two or more sets of the second set of echoes.

For real-time imaging, the echoes are acquired continuously, and for each new acquisition, one B-mode image and one elastography image (e.g., by buffering the previous data) are generated.

At 1214, an elastogram visualization algorithm is retrieved based on an elastogram mode of operation of interest. As discussed herein, the mode can be a default, user specified, changed, etc.

At 1216, in response to the mode of operation of interest being a soft blend mode, the soft blend 306 algorithm is applied. For this, for a pixel in the strain image, a corresponding pixel in the correlation image is identified. A correlation value is identified for the identified pixel. A transparency level is then identified for the identified correlation value. The transparency level is then applied to the pixel in the strain image. This is repeated for other pixels in the strain image.

At 1218, in response to the mode of operation of interest being a hard blend mode, the hard blend 308 algorithm is applied. For this, for a pixel in the strain image, a corresponding pixel in the correlation image is identified. A correlation value is identified for the identified pixel. The identified correlation pixel value is compared against a predetermined threshold. If the correlation pixel value is less than the threshold, the corresponding strain image pixel is set to completely transparent or ignored. Otherwise, the corresponding strain image pixel is set to semi-transparent or opaque. This is repeated for other pixels in the strain image.

At 1220, in response to the mode of operation of interest being a B-mode priority, the B-mode priority 310 algorithm is applied. For this, for a pixel in the B-mode image, a pixel is identified. The identified B-mode image pixel value is compared against a predetermined threshold. If the B-mode image pixel value is less than the threshold, the corresponding strain image pixel is set to completely transparent or ignored. Otherwise, the corresponding strain image pixel is set to semi-transparent or opaque. This is repeated for other pixels in the B-mode image.

At 1222, the modified strain image is displayed, superimposed over the B-mode image.

At least a portion of the method discussed herein may be implemented by way of computer readable instructions, encoded or embedded on computer readable storage medium (which excludes transitory medium), which, when executed by a computer processor(s), causes the processor(s) to carry out the described acts. Additionally or alternatively, at least one of the computer readable instructions is carried by a signal, carrier wave or other transitory medium.

Generally, the embodiments described herein provide only valid and informative elastograms to the end user, for example, by making invalid regions of the strain image slightly or completely transparent, and/or blocking the elastography from displaying regions of the strain image that corresponds to regions of the B-mode image where not enough signal was acquired, for example, dark regions in the B-mode image.

The embodiments can be used, for example, during the training phase when the clinicians are trying to improve their scanning techniques, during live scan to provide feedback to an end user such that the end user knows when a good sequence of elastograms have been acquired, during exam review when selecting individual frames where good images are generated, etc.

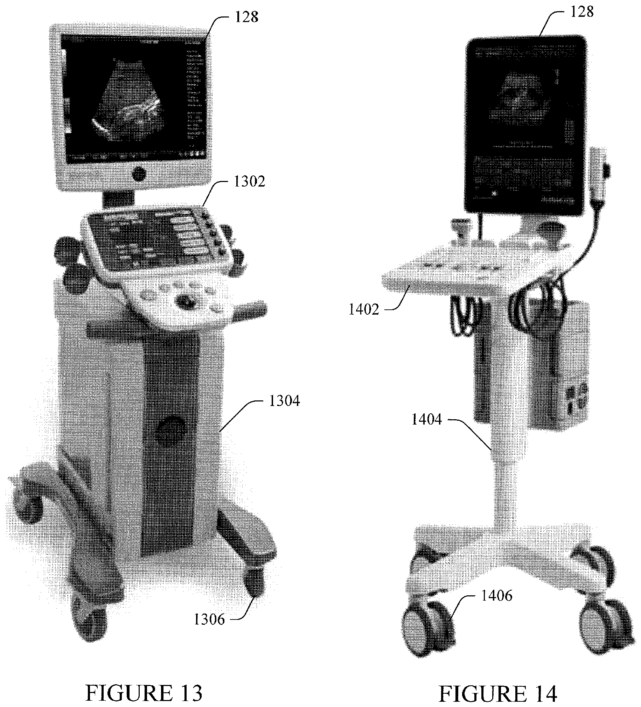

Figures 13 and 14 illustrate non-limiting examples of the ultrasound imaging system 100. In the non-limiting examples, at least the echo processor 116, the elastography processor 118, the scan converter 120, and the rendering engine 122 are part of console 1302 and 1402, and the display 124 and the console 1302 and 1402 are integrated in and part of respective mobile carts 1304 and 1404, which include movers 1306 and 1406 such as wheels, casters, etc.

In another configuration, the ultrasound imaging system 100 does not include movers and/or is not integrated into a cart, but instead rests on a table, desk, etc. In another configuration, the ultrasound imaging system 100 is part of a hand-held ultrasound scanner. An example of a hand-held scanner is described in US patent 7,699,776, entitled "Intuitive Ultrasonic Imaging System and Related Method Thereof," and filed on March 6, 2003.

The application has been described with reference to various embodiments. Modifications and alterations will occur to others upon reading the application. It is intended that the invention be construed as including all such modifications and alterations, including insofar as they come within the scope of the appended claims and the equivalents thereof.

## Claims

1. A method of ultrasound imaging comprising:
transmitting (1202) an ultrasound signal into a field of view in which an object is disposed;
receiving (1204) a first set of echoes from the field of view;
generating (1210) a B-mode image from the received first set of echoes;
applying (1206) compression to the object within the field of view;
receiving (1208) at least one second set of echoes from the field of view, each second set of echoes comprising compression echoes;
generating (1212) a strain image from a displacement image produced by estimating a motion between sequences of the second sets of echoes;
generating (1212) a correlation image indicating a degree of similarity between sequences of the second sets of echoes;
modifying (1216, 1218) pixel values of the strain image based on one of: the degree of similarity between sequences of the second sets of echoes and the generated B-mode image to generate a modified strain image;
displaying the B-mode image; and
superimposing (1222) the modified strain image over the B-mode image.

2. The method of claim 1, further comprising:
modifying the pixel values of the strain image based on at least one of: pixel values of the correlation image and pixel values of the B-mode image to generate the modified strain image.

3. The method of claim 1 or 2, further comprising:
identifying a pixel of the strain image;
identifying a pixel of the correlation image corresponding to the pixel of the strain image;
identifying a pixel value of the identified pixel of the correlation image, the pixel value being a correlation value within a predetermined range;
retrieving a predetermined transparency value for the identified correlation value;
applying the transparency value to the pixel of the strain image to create the modified strain image pixel; and
superimposing the modified strain image pixel over the corresponding B-mode image pixel.

4. The method of claim 3, further comprising:
prior to applying the transparency value, scaling the transparency value as a function of the correlation value of the pixel of the correlation image, and, the step of applying the transparency value to the pixel of the strain image includes applying the scaled transparency value.

5. The method of claim 4, wherein scaling the transparency value comprises at least one of: a continuous transition in transparency and a discrete transition in transparency.

6. The method of claim 4 or 5, wherein scaling the transparency value comprises at least one of: a linear transition in transparency and a non-linear transition in transparency.

7. The method of claim 3, wherein the step of applying the transparency value to the pixel of the strain image to create the modified strain image pixel comprises applying a transparency value which is either transparent or in a range from semi-transparent to opaque.

8. The method of any one of claims 3 to 7, further comprising:
retrieving the transparency value from a look up table mapping transparency values to correlation values.

9. The method of claim 1, further comprising:
identifying a pixel of the strain image;
identifying a pixel of the correlation image corresponding to the pixel of the strain image;
identifying a pixel value of the identified pixel of the correlation image, the pixel value being a correlation value within a predetermined range;
comparing the identified pixel value with a predetermined threshold;
rendering the pixel of the strain image completely transparent in response to the identified pixel value not satisfying the predetermined threshold to create the modified strain image pixel;
rendering the pixel of the strain image semi-transparent or opaque in response to the identified pixel value satisfying the predetermined threshold to create the modified strain image pixel; and
superimposing the modified strain image pixel over the corresponding B-mode image pixel.

10. The method of claim 9, further comprising:
receiving a signal indicative of a user change in the predetermined threshold to create an updated threshold;
comparing the identified pixel value with the updated threshold;
rendering the pixel of the strain image completely transparent in response to the identified pixel value not satisfying the updated threshold to create the modified strain image pixel;
rendering the pixel of the strain image semi-transparent or opaque in response to the identified pixel value satisfying the updated threshold to create the modified strain image pixel; and
superimposing the modified strain image pixel over the corresponding B-mode image pixel.

11. The method of claim 1, further comprising:
identifying a pixel of the B-mode image;
identifying a pixel value for the identified pixel of the B-mode image;
comparing the identified pixel value with a predetermined threshold;
rendering a pixel of the strain image corresponding to the pixel of the B-mode image completely transparent in response to the identified pixel value not satisfying the predetermined threshold to create the modified strain image pixel;
rendering the pixel of the strain image corresponding to the pixel of the B-mode image semi-transparent or opaque in response to the identified pixel value satisfying the predetermined threshold to create the modified strain image pixel; and
superimposing the modified strain image pixel over the corresponding B-mode image pixel.

12. An ultrasound imaging system (100) comprising:
a transducer array (102, 104) configured for transmitting ultrasound signals into a field of view and for receiving echoes from the field of view, the received echoes including a first set of echoes and at least a second set of echoes, each second set of echoes comprising compression echoes;
transmit circuitry (106) configured for generating the ultrasound signals for transmission by the transducer array (102, 104);
receive circuitry (108) configured for receiving the echoes from the transducer array (102, 104);
a controller (112) configured for controlling the transmit circuitry (106) and the receive circuitry (108);
an echo processor (116) configured for receiving the first set of echoes from the receive circuitry (108) and for processing the first set of echoes to generate a B-mode image;
an elastogram processor (118) configured for receiving the at least one second set of echoes from the receive circuitry (108) and for processing the at least one second set of echoes for:
estimating motion between sequences of the second sets of echoes;
generating a displacement image from the estimated motion;
generating a correlation image indicating a degree of similarity between sequences of the second sets of echoes;
generating a strain image from a displacement image produced by estimating motion; and
modifying pixel values of the strain image based on one of: the degree of similarity between sequences of the second sets of echoes and the generated B-mode image to generate a modified strain image; and
a rendering engine (122) configured for receiving data for display from the echo processor (116) and the elastogram processor (118) and for processing the received data for displaying the B-mode image and for superimposing the modified strain image over the B-mode image.

13. The system according to claim 12, wherein the rendering engine (122) is further configured for:
identifying a pixel of the strain image;
identifying a pixel of the correlation image corresponding to the pixel of the strain image;
identifying a pixel value of the identified pixel of the correlation image, the pixel value being a correlation value within a predetermined range;
retrieving a predetermined transparency value for the identified correlation value;
applying the transparency value to the pixel of the strain image to create the moduified strain image pixel; and
superimposing the modified strain image pixel over the corresponding B-mode image pixel.

14. The system according to claim 12, wherein the rendering engine (122) is further configured for:
identifying a pixel of the strain image;
identifying a pixel of the correlation image corresponding to the pixel of the strain image;
identifying a pixel value of the identified pixel of the correlation image, the pixel value being a correlation value within a predetermined range;
comparing the identified pixel value with a predetermined threshold;
rendering the pixel of the strain image completely transparent in response to the identified pixel value not satisfying the predetermined threshold to create the modified strain image pixel;
rendering the pixel of the strain image semi-transparent or opaque in response to the identified pixel value satisfying the predetermined threshold to create the modified strain image pixel; and
superimposing the modified strain image pixel over the corresponding B-mode image pixel.

15. The system according to claim 12, wherein the rendering engine (122) is further configured for:
identifying a pixel of the strain image;
identifying a pixel of the correlation image corresponding to the pixel of the strain image;
identifying a pixel value of the identified pixel of the correlation image, the pixel value being a correlation value within a predetermined range;
comparing the identified pixel value with a predetermined threshold;
rendering the pixel of the strain image completely transparent in response to the identified pixel value not satisfying the predetermined threshold to create the modified strain image pixel;
rendering the pixel of the strain image semi-transparent or opaque in response to the identified pixel value satisfying the predetermined threshold to create the modified strain image pixel; and
superimposing the modified strain image pixel over the corresponding B-mode image pixel.

## Patentansprüche

1. Ultraschallbildgebungsverfahren, umfassend:
Übertragen (1202) eines Ultraschallsignals in ein Sichtfeld, in dem ein Objekt angeordnet ist;
Empfangen (1204) eines ersten Echosatzes vom Sichtfeld;
Erzeugen (1210) eines B-Modus-Bildes vom empfangenen ersten Echosatz;
Anwenden (1206) von Kompression auf das Objekt innerhalb des Sichtfeldes;
Empfangen (1208) mindestens eines zweiten Echosatzes vom Sichtfeld, wobei jeder zweite Echosatz Kompressionsechos umfasst;
Erzeugen (1212) eines Dehnungsbildes von einem Verschiebungsbild, das durch Schätzen einer Bewegung zwischen Sequenzen der zweiten Echosätze produziert wird;
Erzeugen (1212) eines Korrelationsbildes, das einen Ähnlichkeitsgrad zwischen Sequenzen der zweiten Echosätze angibt;
Modifizieren (1216, 1218) von Pixelwerten des Dehnungsbildes basierend auf einem von: dem Ähnlichkeitsgrad zwischen Sequenzen der zweiten Echosätze und des erzeugten B-Modus-Bildes, um ein modifiziertes Dehnungsbild zu erzeugen;
Anzeigen des B-Modus-Bildes; und
Überlagern (1222) des modifizierten Dehnungsbildes über das B-Modus-Bild.

2. Verfahren nach Anspruch 1, weiter umfassend:
Modifizieren der Pixelwerte des Dehnungsbildes basierend auf mindestens einem von: Pixelwerten des Korrelationsbildes und Pixelwerten des B-Modus-Bildes, um das modifizierte Dehnungsbild zu erzeugen.

3. Verfahren nach Anspruch 1 oder 2, weiter umfassend:
Identifizieren eines Pixels des Dehnungsbildes;
Identifizieren eines Pixels des Korrelationsbildes, das dem Pixel des Dehnungsbildes entspricht;
Identifizieren eines Pixelwerts des identifizierten Pixels des Korrelationsbildes, wobei der Pixelwert ein Korrelationswert innerhalb eines vorbestimmten Bereichs ist;
Abrufen eines vorbestimmten Transparenzwerts für den identifizierten Korrelationswert;
Anwenden des Transparenzwerts an das Pixel des Dehnungsbildes, um das modifizierte Dehnungsbild-Pixel zu erstellen; und
Überlagern des modifizierten Dehnungsbild-Pixels über das entsprechende B-Modus-Bild-Pixel.

4. Verfahren nach Anspruch 3, weiter umfassend:
vor Anwenden des Transparenzwerts, Skalieren des Transparenzwerts als eine Funktion des Korrelationswerts des Pixels des Korrelationsbildes, und der Schritt des Anwendens des Transparenzwerts an das Pixel des Dehnungsbildes das Anwenden des skalierten Transparenzwerts einschließt.

5. Verfahren nach Anspruch 4, wobei das Skalieren des Transparenzwerts mindestens eines umfasst von: einem kontinuierlichen Transparenzübergang und einem diskreten Transparenzübergang.

6. Verfahren nach Anspruch 4 oder 5, wobei das Skalieren des Transparenzwerts mindestens eines umfasst von: einem linearen Transparenzübergang und einem nicht linearen Transparenzübergang.

7. Verfahren nach Anspruch 3, wobei der Schritt des Anwendens des Transparenzwerts an das Pixel des Dehnungsbildes, um das modifizierte Dehnungsbild-Pixel zu erstellen, das Anwenden eines Transparenzwerts umfasst, der entweder transparent oder in einem Bereich von halbtransparent bis undurchsichtig ist.

8. Verfahren nach einem der Ansprüche 3 bis 7, weiter umfassend:
Abrufen des Transparenzwerts von einer Nachschlagetabelle, die Transparenzwerte auf Korrelationswerte abbildet.

9. Verfahren nach Anspruch 1, weiter umfassend:
Identifizieren eines Pixels des Dehnungsbildes;
Identifizieren eines Pixels des Korrelationsbildes, das dem Pixel des Dehnungsbildes entspricht;
Identifizieren eines Pixelwerts des identifizierten Pixels des Korrelationsbildes, wobei der Pixelwert ein Korrelationswert innerhalb eines vorbestimmten Bereichs ist;
Vergleichen des identifizierten Pixelwerts mit einem vorbestimmten Schwellenwert; vollständiges Transparentmachen des Pixels des Dehnungsbildes als Antwort darauf, dass der identifizierte Pixelwert den vorbestimmten Schwellenwert nicht erfüllt, um das modifizierte Dehnungsbild-Pixel zu erstellen;
Halbtransparent- oder Undurchsichtigmachen des Pixels des Dehnungsbildes als Antwort darauf, dass der identifizierte Pixelwert den vorbestimmten Schwellenwert erfüllt, um das modifizierte Dehnungsbild-Pixel zu erstellen; und
Überlagern des modifizierten Dehnungsbild-Pixels über das entsprechende B-Modus-Bild-Pixel.

10. Verfahren nach Anspruch 9, weiter umfassend:
Empfangen eines Signals, das eine Benutzeränderung des vorbestimmten Schwellenwerts angibt, um einen aktualisierten Schwellenwert zu erstellen;
Vergleichen des identifizierten Pixelwerts mit dem aktualisierten Schwellenwert;
vollständiges Transparentmachen des Pixels des Dehnungsbildes als Antwort darauf, dass der identifizierte Pixelwert den aktualisierten Schwellenwert nicht erfüllt, um das modifizierte Dehnungsbild-Pixel zu erstellen;
Halbtransparent- oder Undurchsichtigmachen des Pixels des Dehnungsbildes als Antwort darauf, dass der identifizierte Pixelwert den aktualisierten Schwellenwert erfüllt, um das modifizierte Dehnungsbild-Pixel zu erstellen; und
Überlagern des modifizierten Dehnungsbild-Pixels über das entsprechende B-Modus-Bild-Pixel.

11. Verfahren nach Anspruch 1, weiter umfassend:
Identifizieren eines Pixels des B-Modus-Bildes;
Identifizieren eines Pixelwerts für das identifizierte Pixel des B-Modus-Bildes;
Vergleichen des identifizierten Pixelwerts mit einem vorbestimmten Schwellenwert;
vollständiges Transparentmachen eines Pixels des Dehnungsbildes, das dem Pixel des B-Modus-Bildes entspricht, als Antwort darauf, dass der identifizierte Pixelwert den vorbestimmten Schwellenwert nicht erfüllt, um das modifizierte Dehnungsbild-Pixel zu erstellen;
Halbtransparent- oder Undurchsichtigmachen des Pixels des Dehnungsbildes, das dem Pixel des B-Modus-Bildes entspricht, als Antwort darauf, dass der identifizierte Pixelwert den vorbestimmten Schwellenwert erfüllt, um das modifizierte Dehnungsbild-Pixel zu erstellen; und
Überlagern des modifizierten Dehnungsbild-Pixels über das entsprechende B-Modus-Bild-Pixel.

12. Ultraschallbildgebungssystem (100), umfassend:
ein Wandler-Array (102, 104), das konfiguriert ist, um Ultraschallsignale in ein Sichtfeld zu übertragen und um Echos vom Sichtfeld zu empfangen, wobei die empfangenen Echos einen ersten Echosatz und mindestens einen zweiten Echosatz einschließen, wobei jeder zweite Echosatz Kompressionsechos umfasst;
Übertragungsschaltkreise (106), die konfiguriert sind, um die Ultraschallsignale für eine Übertragung durch das Wandler-Array (102, 104) zu erzeugen;
Empfangsschaltkreise (108), die konfiguriert sind, um die Echos vom Wandler-Array (102, 104) zu empfangen;
eine Steuerung (112), die konfiguriert ist, um die Übertragungsschaltkreise (106) und die Empfangsschaltkreise (108) zu steuern;
einen Echoprozessor (116), der konfiguriert ist, um den ersten Echosatz von den Empfangsschaltkreisen (108) zu empfangen und um den ersten Echosatz zu verarbeiten, um ein B-Modus-Bild zu erzeugen;
einen Elastogrammprozessor (118), der konfiguriert ist, um den mindestens einen zweiten Echosatz von den Empfangsschaltkreisen (108) zu empfangen und um den mindestens einen zweiten Echosatz zu verarbeiten, um:
eine Bewegung zwischen Sequenzen der zweiten Echosätze zu schätzen;
ein Verschiebungsbild von der geschätzten Bewegung zu erzeugen;
ein Korrelationsbild, das einen Ähnlichkeitsgrad zwischen Sequenzen der zweiten Echosätze angibt, zu erzeugen;
ein Dehnungsbild von einem Verschiebungsbild, das durch Schätzen einer Bewegung produziert wird, zu erzeugen; und
Pixelwerte des Dehnungsbildes zu modifizieren, basierend auf einem von: dem Ähnlichkeitsgrad zwischen Sequenzen der zweiten Echosätze und des erzeugten B-Modus-Bildes, um ein modifiziertes Dehnungsbild zu erzeugen; und
eine Rendering-Engine (122), die konfiguriert ist, um Daten zur Anzeige von dem Echoprozessor (116) und dem Elastogrammprozessor (118) zu empfangen und um die empfangenen Daten zum Anzeigen des B-Modus-Bildes zu verarbeiten und um das modifizierte Dehnungsbild über das B-Modus-Bild zu überlagern.

13. System nach Anspruch 12, wobei die Rendering-Engine (122) weiter konfiguriert ist, um:
ein Pixel des Dehnungsbildes zu identifizieren;
ein Pixel des Korrelationsbildes, das dem Pixel des Dehnungsbildes entspricht, zu identifizieren;
einen Pixelwert des identifizierten Pixels des Korrelationsbildes zu identifizieren, wobei der Pixelwert ein Korrelationswert innerhalb eines vorbestimmten Bereichs ist;
einen vorbestimmten Transparenzwert für den identifizierten Korrelationswert abzurufen;
den Transparenzwert an das Pixel des Dehnungsbildes anzuwenden, um das modifizierte Dehnungsbild-Pixel zu erstellen; und
das modifizierte Dehnungsbild-Pixel über das entsprechende B-Modus-Bild-Pixel zu überlagern.

14. System nach Anspruch 12, wobei die Rendering-Engine (122) weiter konfiguriert ist, um:
ein Pixel des Dehnungsbildes zu identifizieren;
ein Pixel des Korrelationsbildes, das dem Pixel des Dehnungsbildes entspricht, zu identifizieren;
einen Pixelwert des identifizierten Pixels des Korrelationsbildes zu identifizieren, wobei der Pixelwert ein Korrelationswert innerhalb eines vorbestimmten Bereichs ist;
den identifizierten Pixelwert mit einem vorbestimmten Schwellenwert zu vergleichen;
das Pixel des Dehnungsbildes vollständig transparent zu machen als Antwort darauf, dass der identifizierte Pixelwert den vorbestimmten Schwellenwert nicht erfüllt, um das modifizierte Dehnungsbild-Pixel zu erstellen;
das Pixel des Dehnungsbildes halbtransparent oder undurchsichtig zu machen als Antwort darauf, dass der identifizierte Pixelwert den vorbestimmten Schwellenwert erfüllt, um das modifizierte Dehnungsbild-Pixel zu erstellen; und
das modifizierte Dehnungsbild-Pixel über das entsprechende B-Modus-Bild-Pixel zu überlagern.

15. System nach Anspruch 12, wobei die Rendering-Engine (122) weiter konfiguriert ist, um:
ein Pixel des Dehnungsbildes zu identifizieren;
ein Pixel des Korrelationsbildes, das dem Pixel des Dehnungsbildes entspricht, zu identifizieren;
einen Pixelwert des identifizierten Pixels des Korrelationsbildes zu identifizieren, wobei der Pixelwert ein Korrelationswert innerhalb eines vorbestimmten Bereichs ist;
den identifizierten Pixelwert mit einem vorbestimmten Schwellenwert zu vergleichen;
das Pixel des Dehnungsbildes vollständig transparent zu machen als Antwort darauf, dass der identifizierte Pixelwert den vorbestimmten Schwellenwert nicht erfüllt, um das modifizierte Dehnungsbild-Pixel zu erstellen;
das Pixel des Dehnungsbildes halbtransparent oder undurchsichtig zu machen als Antwort darauf, dass der identifizierte Pixelwert den vorbestimmten Schwellenwert erfüllt, um das modifizierte Dehnungsbild-Pixel zu erstellen; und
das modifizierte Dehnungsbild-Pixel über das entsprechende B-Modus-Bild-Pixel zu überlagern.

## Revendications

1. Procédé d'imagerie par ultrasons comprenant :
l'émission (1202) d'un signal ultrasonore dans un champ de vision dans lequel un objet est disposé ;
la réception (1204) d'un premier ensemble d'échos provenant du champ de vision ;
la génération (1210) d'une image en mode B à partir du premier ensemble d'échos reçu ;
l'application (1206) d'une compression à l'objet au sein du champ de vision ;
la réception (1208) d'au moins un second ensemble d'échos provenant du champ de vision, chaque second ensemble d'échos comprenant des échos de compression ;
la génération (1212) d'une image de déformation à partir d'une image de déplacement produite par l'estimation d'un mouvement entre des séquences des seconds ensembles d'échos ;
la génération (1212) d'une image de corrélation indiquant un degré de similarité entre des séquences des seconds ensemble d'échos ;
la modification (1216, 1218) de valeurs de pixel de l'image de déformation sur la base de l'un parmi : le degré de similarité entre des séquences des seconds ensembles d'échos et l'image en mode B générée pour générer une image de déformation modifiée ;
l'affichage de l'image en mode B ; et
la superposition (1222) de l'image de déformation modifiée sur l'image en mode B.

2. Procédé selon la revendication 1, comprenant en outre :
la modification des valeurs de pixel de l'image de déformation sur la base d'au moins l'un parmi : des valeurs de pixel de l'image de corrélation et des valeurs de pixel de l'image en mode B pour générer l'image de déformation modifiée.

3. Procédé selon la revendication 1 ou 2, comprenant en outre :
l'identification d'un pixel de l'image de déformation ;
l'identification d'un pixel de l'image de corrélation correspondant au pixel de l'image de déformation ;
l'identification d'une valeur de pixel du pixel identifié de l'image de corrélation, la valeur de pixel étant une valeur de corrélation au sein d'une plage prédéterminée ;
la récupération d'une valeur de transparence prédéterminée pour la valeur de corrélation identifiée ;
l'application de la valeur de transparence au pixel de l'image de déformation pour créer le pixel d'image de déformation modifié ; et
la superposition du pixel d'image de déformation modifié sur le pixel d'image en mode B correspondant.

4. Procédé selon la revendication 3, comprenant en outre :
avant l'application de la valeur de transparence, la mise à l'échelle de la valeur de transparence en fonction de la valeur de corrélation du pixel de l'image de corrélation, et, l'étape d'application de la valeur de transparence au pixel de l'image de déformation inclut l'application de la valeur de transparence mise à l'échelle.

5. Procédé selon la revendication 4, dans lequel la mise en échelle de la valeur de transparence comprend au moins l'un parmi : une transition continue de transparence et une transition discrète de transparence.

6. Procédé selon la revendication 4 ou 5, dans lequel la mise à l'échelle de la valeur de transparence comprend au moins l'un parmi : une transition linéaire de transparence et une transition non linéaire de transparence.

7. Procédé selon la revendication 3, dans lequel l'étape d'application de la valeur de transparence au pixel de l'image de déformation pour créer le pixel d'image de déformation modifié comprend l'application d'une valeur de transparence qui est soit transparente, soit dans une plage allant de semi transparent à opaque.

8. Procédé selon l'une quelconque des revendications 3 à 7, comprenant en outre :
la récupération de la valeur de transparence à partir d'une table de conversion établissant une correspondance entre les valeurs de transparence et les valeurs de corrélation.

9. Procédé selon la revendication 1, comprenant en outre :
l'identification d'un pixel de l'image de déformation ;
l'identification d'un pixel de l'image de corrélation correspondant au pixel de l'image de déformation ;
l'identification d'une valeur de pixel du pixel identifié de l'image de corrélation, la valeur de pixel étant une valeur de corrélation au sein d'une plage prédéterminée ;
la comparaison de la valeur de pixel identifiée à un seuil prédéterminé ;
le fait de rendre le pixel de l'image de déformation complètement transparent en réponse à la valeur de pixel identifiée ne satisfaisant pas le seuil prédéterminé pour créer le pixel d'image de déformation modifié ;
le fait de rendre le pixel de l'image de déformation semi-transparent ou opaque en réponse à la valeur de pixel identifiée satisfaisant le seuil prédéterminé pour créer le pixel d'image de déformation modifié ; et
la superposition du pixel d'image de déformation modifié sur le pixel d'image en mode B correspondant.

10. Procédé selon la revendication 9, comprenant en outre :
la réception d'un signal indiquant un changement par un utilisateur du seuil prédéterminé pour créer un seuil actualisé ;
la comparaison de la valeur de pixel identifiée au seuil actualisé ;
le fait de rendre le pixel de l'image de déformation complètement transparent en réponse à la valeur de pixel identifiée ne satisfaisant pas le seuil actualisé pour créer le pixel d'image de déformation modifié ;
le fait de rendre le pixel de l'image de déformation semi-transparent ou opaque en réponse à la valeur de pixel identifiée satisfaisant le seuil actualisé pour créer le pixel d'image de déformation modifié ; et
la superposition du pixel d'image de déformation modifié sur le pixel d'image en mode B correspondant.

11. Procédé selon la revendication 1, comprenant en outre :
l'identification d'un pixel de l'image en mode B ;
l'identification d'une valeur de pixel pour le pixel identifié de l'image en mode B ;
la comparaison de la valeur de pixel identifiée à un seuil prédéterminé ;
le fait de rendre un pixel de l'image de déformation correspondant au pixel de l'image en mode B complètement transparent en réponse à la valeur de pixel identifiée ne satisfaisant pas le seuil prédéterminé pour créer le pixel d'image de déformation modifié ;
le fait de rendre le pixel de l'image de déformation correspondant au pixel de l'image en mode B semi-transparent ou opaque en réponse à la valeur de pixel identifiée satisfaisant le seuil prédéterminé pour créer le pixel d'image de déformation modifié ; et
la superposition du pixel d'image de déformation modifié sur le pixel d'image en mode B correspondant.

12. Système d'imagerie par ultrasons (100) comprenant :
un réseau de transducteurs (102, 104) configuré pour émettre des signaux ultrasonores dans un champ de vision et pour recevoir des échos provenant du champ de vision, les échos reçus incluant un premier ensemble d'échos et au moins un second ensemble d'échos, chaque second ensemble d'échos comprenant des échos de compression ;
un circuit d'émission (106) configuré pour générer les signaux ultrasonores à des fins d'émission par le réseau de transducteurs (102, 104) ;
un circuit de réception (108) configuré pour recevoir les échos à partir du réseau de transducteurs (102, 104) ;
un dispositif de commande (112) configuré pour commander le circuit d'émission (106) et le circuit de réception (108) ;
un processeur d'écho (116) configuré pour recevoir le premier ensemble d'échos à partir du circuit de réception (108) et pour traiter le premier ensemble d'échos pour générer une image en mode B ;
un processeur d'élastogramme (118) configuré pour recevoir l'au moins un second ensemble d'échos à partir du circuit de réception (108) et pour traiter l'au moins un second ensemble d'échos pour :
estimer un mouvement entre des séquences des seconds ensembles d'échos ;
générer une image de déplacement à partir du mouvement estimé ;
générer une image de corrélation indiquant un degré de similarité entre des séquences des seconds ensembles d'échos ;
générer une image de déformation à partir d'une image de déplacement produite par l'estimation d'un mouvement ; et
modifier des valeurs de pixel de l'image de déformation sur la base de l'un parmi : le degré de similarité entre des séquences des seconds ensembles d'échos et l'image en mode B générée pour générer une image de déformation modifiée ; et
un moteur de rendu (122) configuré pour recevoir des données pour affichage à partir du processeur d'écho (116) et du processeur d'élastogramme (118) et pour traiter les données reçues pour afficher l'image en mode B et pour superposer l'image de déformation modifiée sur l'image en mode B.

13. Système selon la revendication 12, dans lequel le moteur de rendu (122) est configuré en outre pour :
identifier un pixel de l'image de déformation ;
identifier un pixel de l'image de corrélation correspondant au pixel de l'image de déformation ;
identifier une valeur de pixel du pixel identifié de l'image de corrélation, la valeur de pixel étant une valeur de corrélation au sein d'une plage prédéterminée ;
récupérer une valeur de transparence prédéterminée pour la valeur de corrélation identifiée ;
appliquer la valeur de transparence au pixel de l'image de déformation pour créer le pixel d'image de déformation modifié ; et
superposer le pixel d'image de déformation modifié sur le pixel d'image en mode B correspondant.

14. Système selon la revendication 12, dans lequel le moteur de rendu (122) est configuré en outre pour :
identifier un pixel de l'image de déformation ;
identifier un pixel de l'image de corrélation correspondant au pixel de l'image de déformation ;
identifier une valeur de pixel du pixel identifié de l'image de corrélation, la valeur de pixel étant une valeur de corrélation au sein d'une plage prédéterminée ;
comparer la valeur de pixel identifiée à un seuil prédéterminé ;
rendre le pixel de l'image de déformation complètement transparent en réponse à la valeur de pixel identifiée ne satisfaisant pas le seuil prédéterminé pour créer le pixel d'image de déformation modifié ;
rendre le pixel de l'image de déformation semi-transparent ou opaque en réponse à la valeur de pixel identifiée satisfaisant le seuil prédéterminé pour créer le pixel d'image de déformation modifié ; et
superposer le pixel d'image de déformation modifié sur le pixel d'image en mode B correspondant.

15. Système selon la revendication 12, dans lequel le moteur de rendu (122) est configuré en outre pour :
identifier un pixel de l'image de déformation ;
identifier un pixel de l'image de corrélation correspondant au pixel de l'image de déformation ;
identifier une valeur de pixel du pixel identifié de l'image de corrélation, la valeur de pixel étant une valeur de corrélation au sein d'une plage prédéterminée ;
comparer la valeur de pixel identifiée à un seuil prédéterminé ;
rendre le pixel de l'image de déformation complètement transparent en réponse à la valeur de pixel identifiée ne satisfaisant pas le seuil prédéterminé pour créer le pixel d'image de déformation modifié ;
rendre le pixel de l'image de déformation semi-transparent ou opaque en réponse à la valeur de pixel identifiée satisfaisant le seuil prédéterminé pour créer le pixel d'image de déformation modifié ; et
superposer le pixel d'image de déformation modifié sur le pixel d'image en mode B correspondant.
